# EUROPEAN PATENT APPLICATION

(11) **EP 4 378 947 A1**
(43) Date of publication of application: **05.06.2024**
(21) Application number: 22849383.9
(22) Date of filing: 22.07.2022
(51) Int. Cl.: C07K 2/00

(54) **METHOD FOR IDENTIFYING SPLICING PEPTIDE**

(30) Priority: 28.07.2021 US 202163226406 P
(71) Applicant: Providence Health & Services - Oregon, Portland, OR 97213 (US); Shimadzu Corporation, Nakagyo-ku, Kyoto-shi, Kyoto 604-8511 (JP)
(72) Inventor: KOGUCHI, Yoshinobu, Portland, Oregon 97213 (US); IWAMOTO, Noriko, Columbia, Maryland 21046 (US); SHIMADA, Takashi, Columbia, Maryland 21046 (US)
(74) Representative: Müller-Boré & Partner Patentanwälte PartG mbB
(86) International application number: PCT/JP2022/028431
(87) International publication number: WO 2023/008321

(57) **Abstract**

A method of identifying an unknown spliced peptide derived from a known parent peptide contained in a biological sample, the method comprising: (1) a first step involving performing mass spectrometry on the sample to obtain mass spectrometry data regarding a peptide contained in the sample; (2) a second step involving searching a primary library, which is a database of known amino acid sequences, for an amino acid sequence that matches the mass spectrometry data, to identify a parent peptide contained in the sample; (3) a third step involving creating a secondary library including a candidate group of spliced peptides obtainable from the identified parent peptide; and (4) a fourth step involving searching the secondary library for an amino acid sequence that matches the mass spectrometry data, to identify a spliced peptide contained in the sample.

## Description

### TECHNICAL FIELD

The present invention relates to a method of identifying a spliced peptide.

### BACKGROUND ART

The immune system recognizes self from non-self in order to protect self, and this requires a complex process. The immune system has two mechanisms, innate immunity and adaptive immunity. The main function of innate immunity is to promptly recognize a foreign body and eliminate it. Innate immunity is complemented by the adaptive immune system, which is complex.

The adaptive immune system works as follows. When a foreign body enters a living body, or when a foreign body such as a cancer cell occurs inside a living body, the foreign body is first digested (degraded) by macrophages and the like, and then the degraded foreign body is recognized and captured by dendritic cells. This cell is called an antigen-presenting cell.

The foreign body (a protein) captured by the dendritic cell is transported to the inside of the cell, where it is degraded into peptides by lysosomes, proteasomes, and the like in the cytoplasm. The peptide binds to a major histocompatibility complex (MHC) molecule inside the cell, and in that state, the peptide is presented to lymphocytes (T cells and B cells) and recognized as an antigen by lymphocytes. MHC in humans is called human leukocyte antigen (HLA).

The functions of lymphocytes (T cells and B cells) are cytotoxic immunity (T cells) and humoral immunity (B cells). Mature T cells have an ability to specifically attack a foreign body by recognizing an antigen and using it as a guide. B cells have an ability to produce an antibody capable of binding an antigen they recognized, and thereby neutralize and/or inactivate the foreign body (the antigen).

In recent years, cancer immunotherapy has been receiving attention for its potential to enhance efficacy of cancer therapy (such as surgery, chemotherapy, and radiotherapy). As a cancer immunotherapy, a method called peptide vaccine therapy is known, which involves inoculating a cancer-specific peptide (a peptide only found in cancer cells, not in normal cells) as a vaccine so as to activate the adaptive immunity function to achieve an enhanced therapeutic efficacy for cancer.

HLA genotype (haplotypes) is defined by a pair of loci, one inherited from the mother and one from the father, and it is said that there are tens of thousands of different haplotypes. Due to this diversity of the haplotypes, there are diverse HLA phenotypes (HLA types). It is known that different HLA types bind different HLA-binding peptides (which are peptides capable of binding to HLA; an antigen presented by antigen-presenting cells).

Because of this diversity, in the event of inoculation of a given peptide vaccine, whether the peptide can bind to HLA and then be presented as an antigen for lymphocytes to activate the adaptive immune function may depend on the HLA type of the patient. For this reason, it is desirable that the peptide for use as a vaccine in cancer vaccine therapy be appropriately selected in accordance with the HLA type of the patient.

By the way, it is conventionally believed that the protein splicing takes place at the time of editing of mRNA transcribed from template DNA, but in recent years, it is discovered that, during proteasomal degradation of a foreign protein, reaction such as substitution in the peptide sequence (peptide splicing reaction) occurs (see PTL 1 (European Patent Laying-Open No. 2362225), for example). This peptide splicing reaction may possibly be further increasing the diversity of antigenic peptides, consequently further increasing the individual variation in immune reaction.

In light of the diverse spliced peptides, the diverse HLA-binding peptides, and the like, researches are conducted trying to identify as many antigenic peptides as possible with accuracy.

### CITATION LIST

### PATENT LITERATURE

[PTL 1] European Patent Laying-Open No. 2362225

### SUMMARY OF INVENTION

### TECHNICAL PROBLEM

For instance, a research is conducted on a method that involves synthesizing a protein containing an antigenic peptide candidate region and then actually subjecting the protein to proteasomal degradation and the like to identify an antigenic peptide including a spliced peptide. This method is capable of acquiring antigen information and checking splicing with reliability, but the throughput is low and efficient identification is difficult to achieve.

An alternative method is known, which involves using unidentified MS/MS spectra found in abundance in mass spectrometry data, performing De novo sequence analysis to identify unknown antigenic peptides including spliced peptides, setting a cut-off value on the amino acid sequences of the identified peptides based on a predetermined identification score (ALC: average local confidence, by Peaks Studio software), and thereby identifying the antigenic peptides. However, the data processing is complex, and efficient identification is difficult to achieve. Also, isobaric amino acids cannot be separated, which is rate-limiting in theory.

It should be noted that identification of spliced peptide sequence candidates for a neoantigen cannot be achieved by sequence prediction or matching that is based on genetically-analyzed information regarding cancer-specific mutation and/or based on gene templates. On the other hand, identification information on antigenic peptides identified by mass spectrometry, peptides identified by De novo sequence analysis, and splice sites thereof, as well as information on their affinity for HLA, and the like have been accumulated in resource database (amino acid sequence database) and used as a prediction engine for predicting affinity between HLA and antigenic peptide and for identifying structure, and the like.

The present invention has been devised to solve the above-described problems of conventional identification methods, and an object is to provide a method capable of identifying an unknown spliced peptide with ease and efficiency.

### SOLUTION TO PROBLEM

The present invention relates to a method of identifying an unknown spliced peptide derived from a known parent peptide contained in a biological sample, the method comprising:
(1) a first step involving performing mass spectrometry on the sample to obtain mass spectrometry data regarding a peptide contained in the sample;
(2) a second step involving searching a primary library, which is a database of known amino acid sequences, for an amino acid sequence that matches the mass spectrometry data, to identify a parent peptide contained in the sample;
(3) a third step involving creating a secondary library including a candidate group of spliced peptides obtainable from the identified parent peptide; and
(4) a fourth step involving searching the secondary library for an amino acid sequence that matches the mass spectrometry data, to identify a spliced peptide contained in the sample.

### ADVANTAGEOUS EFFECTS OF INVENTION

The present invention makes it possible to identify an unknown spliced peptide with ease and efficiency by using, as a secondary library, a candidate group of spliced peptides obtainable from a particular parent peptide identified with the use of a primary library.

### BRIEF DESCRIPTION OF DRAWINGS

Fig. 1 is a flowchart describing a method of identifying a spliced peptide according to an embodiment.
Fig. 2 is a schematic view illustrating a reaction mechanism of peptide splicing via an acyl intermediate.

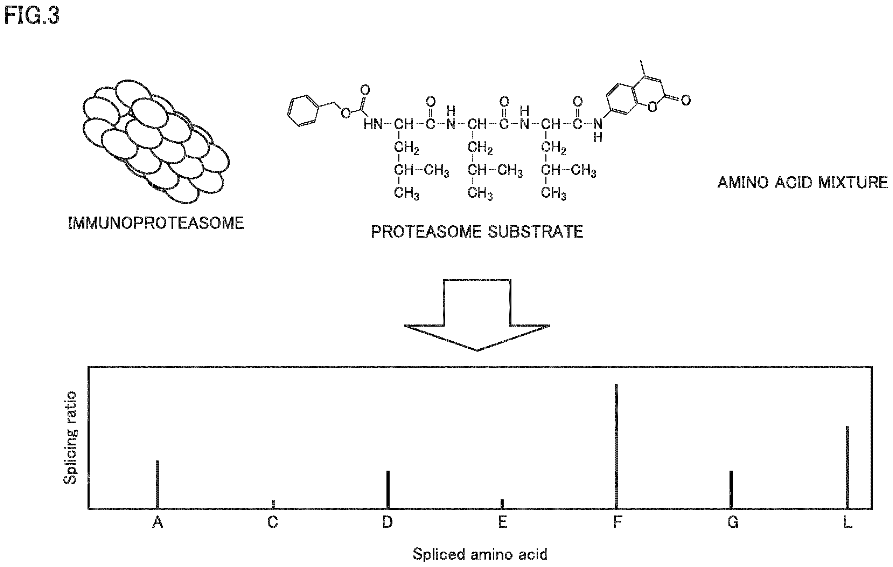

Fig. 3 is a schematic view describing an in vitro test conducted in Reference Example 1 for checking peptide splicing.
Fig. 4 shows the type of an N-terminal amino acid residue of a spliced peptide resulting from S-220.
Fig. 5 shows the type of an N-terminal amino acid residue of a spliced peptide resulting from S-230.
Fig. 6 shows the type of an N-terminal amino acid residue of a spliced peptide resulting from S-260.
Fig. 7 shows the type of an N-terminal amino acid residue of a spliced peptide resulting from S-280.
Fig. 8 shows the type of an N-terminal amino acid residue of a spliced peptide resulting from S-300.
Fig. 9 shows the type of an N-terminal amino acid residue of a spliced peptide resulting from S-310.
Fig. 10 shows the type of an N-terminal amino acid residue of a spliced peptide resulting from S-320.
Fig. 11 shows the type of an N-terminal amino acid residue of a spliced peptide resulting from S-330.

### DESCRIPTION OF EMBODIMENTS

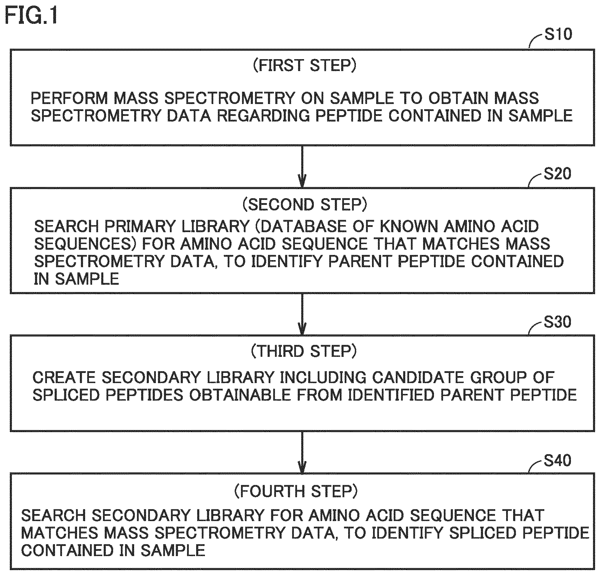

Referring to Fig. 1, a method of identifying a spliced peptide according to the present embodiment is
a method of identifying an unknown spliced peptide derived from a known parent peptide contained in a biological sample, the method comprising:
(1) a first step (S 1) involving performing mass spectrometry on the sample to obtain mass spectrometry data regarding a peptide contained in the sample;
(2) a second step (S2) involving searching a primary library, which is a database of known amino acid sequences, for an amino acid sequence that matches the mass spectrometry data, to identify a parent peptide contained in the sample;
(3) a third step (S3) involving creating a secondary library including a candidate group of spliced peptides obtainable from the identified parent peptide; and
(4) a fourth step (S4) involving searching the secondary library for an amino acid sequence that matches the mass spectrometry data, to identify a spliced peptide contained in the sample.

The identification method according to the present embodiment is a method of identifying an unknown spliced peptide derived from a known parent peptide contained in a biological sample.

The biological sample is, for example, a sample obtained from a living thing. Examples of the sample include a liquid containing a cancer cell, a culture sample of a cancer cell line, isolated cancer tissue, xenograft tissue, blood, and an exosome fraction.

Next, a detailed description will be given of each step of the identification method.

### (First Step: S 1)

In the first step (S 1) according to the present embodiment, mass spectrometry is performed on the sample to obtain mass spectrometry data regarding a peptide contained in the sample.

An example method of the mass spectrometry involves ionizing a protein by matrix-assisted laser desorption ionization (MALDI), electrospray ionization (ESI), or the like and then performing quantitative analysis using the signal intensity of peaks separated based on the mass/charge ratio (m/z) of the ionized protein. The mass spectrometry may also be performed by liquid chromatography-mass spectrometry (LC-MS) and/or liquid chromatography-tandem mass spectrometry (LC-MS/MS).

A mass spectrometer that can be suitably used is a typical single-type mass spectrometer, as well as a tandem mass spectrometer such as a triple quadrupole (QqQ) mass spectrometer, a quadrupole time-of-flight (Q-TOF) mass spectrometer, a tandem time-of-flight (TOF-TOF) mass spectrometer, a quadrupole ion trap (QIT) mass spectrometer, and/or a quadrupole ion trap/time-of-flight (QIT/TOF) mass spectrometer.

At the time of LC-MS/MS, a triple quadrupole mass spectrometer may be used to perform multiple reaction monitoring (MRM). MRM (LC-MS/MS) is an analysis method that is more sensitive than SIM (LC/MS).

The mass spectrometry data may be a mass spectrometry spectrum (such as an MS spectrum and/or an MS/MS spectrum).

### (Second Step: S2)

In the second step (S2) according to the present embodiment, a primary library which is a database (a data set) of known amino acid sequences is searched for an amino acid sequence that matches the mass spectrometry data, to identify a parent peptide contained in the sample.

The parent peptide may be a known HLA-binding peptide (such as HLA Class I-binding peptide and/or HLA Class II-binding peptide).

The parent peptide may be a known peptide derived from a cancer cell (a cancer antigen peptide, a set of predicted sequences of peptides containing a cancer mutation region).

The parent peptide may be a known HLA-binding peptide derived from a cancer cell.

HLA types are broadly classified into two classes (HLA Class I and HLA Class II). The length of a peptide capable of binding to HLA is about 8 to 13 residues for HLA Class I, and about 10 to 25 residues for HLA Class II. HLA Class I is found in almost all cells, and it binds a peptide that is generated from a foreign body (a protein) inside the cell by proteasomal degradation, and presents it as an antigen. HLA Class II binds a peptide that is generated from a foreign body by lysosomal degradation, and presents it as an antigen.

A peptide (an antigen) capable of binding to HLA Class I is directly involved in T cell activation, and therefore such a peptide capable of binding to HLA Class I is very important in cancer therapy.

To date, many resource databases (amino acid sequence databases) have been developed for identification of cancer-specific peptides and the like. Typical examples include web tools such as NetMHCpan (http://www.cbs.dtu.dk/services/NetMHCpan/), IEDB (https://www.iedb.org/), HLA Ligand Atlas (https://hla-ligand-atlas.org/), SysteMHC (https://systemhcatlas.org/), WebLogo (https://weblogo.berkeley.edu/logo.cgi), and HLAthena (http://hlathena.tools/).

When the mass spectrometry data is a mass spectrometry spectrum (such as an MS spectrum and/or an MS/MS spectrum), for example, "the amino acid sequence that matches the mass spectrometry data" refers to an amino acid sequence corresponding to a mass spectrometry spectrum that fits within an acceptable range where a strong correlation with the mass spectrometry spectrum is found by multivariate analysis (such as principal component analysis).

In other words, "the amino acid sequence that matches the mass spectrometry data" means that the mass spectrometry data of the amino acid sequence is simply required to have an acceptable degree of correlation, not necessarily a perfect correlation, with the mass spectrometry data of the sample; in other words, a degree of correlation that is acceptable for amino acid sequence identification conducted based on mass spectrometry data in the relevant technical field. For instance, a certain degree of difference between the mass spectrometry spectra is acceptable that is attributed to a small amount of foreign matter and/or the like that remains in (in other words, that could not be eliminated from) the final sample subjected to mass spectrometry.

The amino acid sequence that matches the mass spectrometry data may be an amino acid sequence corresponding to a mass spectrometry spectrum having a peak at the same m/z value (mass-to-charge ratio) as, or at an m/z value within a predetermined deviation range from, an m/z value for at least one peak in the mass spectrometry spectrum of the sample.

At the time of determining an amino acid sequence that matches the mass spectrometry data, a threshold is used for comparing m/z values, and the absolute deviation between two corresponding m/z values may be set at 0.5 or less, or may be set at 0.2 or less. The m/z value is calculated based on the molecular weight (m) and the state of charge (z; 1, 2, or 3, 4) of the peptide in the mass spectrometry data.

For identifying the amino acid sequence of a peptide, any of various known identification engines may be used, such as Mascot and Peaks Studio. For example, the Mascot system is a protein identification software that enables searching the protein and genome sequence database for an amino acid sequence that matches the peptide mass spectrometry data obtained with a mass spectrometer, and thereby identifying a protein or a peptide contained in the measurement sample. Adopting a probability-based scoring algorithm, it enables score-based distinctive differentiation and visualization of statistically significant proteins or peptides.

### (Third Step: S3)

In the third step (S3) according to the present embodiment, a secondary library including a candidate group of spliced peptides obtainable from the identified parent peptide is created.

The candidate group preferably consists of spliced peptides that are highly likely obtainable from the parent peptide.

This configuration, compared to when all the variations of variant peptides obtainable from the parent peptide through splicing are fed to the candidate group, enables reducing the computer processing time for identification to a realistic time frame, and thereby reducing the cost of identification.

The candidate group preferably consists of variant peptides to be obtained from the parent peptide through at least one of the following: deletion of a predetermined number or less of amino acid residues from at least one of the C-terminus and the N-terminus; and addition of a predetermined number or less of amino acid residues to the C-terminus and the N-terminus. In this configuration, the candidate group also includes variant peptides to be obtained from a "substitution" where the number of added amino acid residues is the same as the number of deleted amino acid residues.

In this configuration, the candidate group consists of spliced peptides that are highly likely obtainable from the parent peptide.

The candidate group may consist of either all of, or part of, the variations of variant peptides to be obtained from the parent peptide through at least one of the following: deletion of a predetermined number or less of amino acid residues from at least one of the C-terminus and the N-terminus; and addition of a predetermined number or less of amino acid residues to the C-terminus and the N-terminus..

The above-described predetermined number for residues is preferably two. More specifically, the candidate group preferably consists of variant peptides to be obtained from the parent peptide through at least one of the following: deletion of two or less amino acid residues from the C-terminus and the N-terminus; and addition of two or less amino acid residues to the C-terminus and the N-terminus (see Table 1 below).

The candidate group in this configuration consists of spliced peptides that are even more highly likely obtainable from the parent peptide, and therefore this configuration enables further reducing the computer processing time for identification, and thereby further reducing the cost of identification.

Conventional methods for identifying neoantigens such as unknown spliced peptides rely on mass spectrometry data such as MS (mass spectrometry) spectra and MS/MS (tandem mass spectrometry) spectra, and involve acquiring mass spectrometry data for many antigenic peptides to identify the amino acid sequences. For the identification of amino acid sequences, sequence database matching or De novo sequence analysis are basically used.

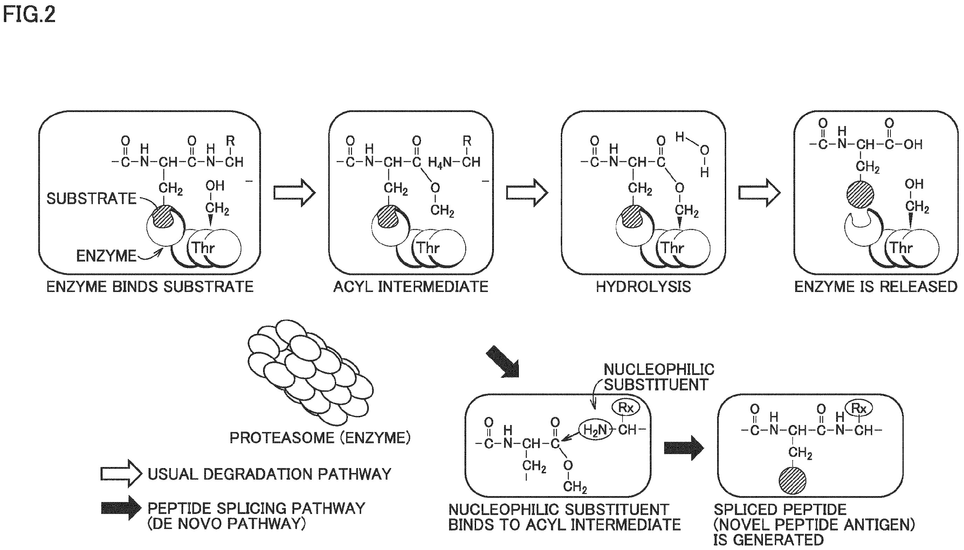

In contrast, the inventors of the present invention have focused their attention on the reaction mechanism of peptide splicing. It is considered that, as shown in Fig. 2, peptide splicing is a reaction where, in the midway through the pathway toward hydrolysis, an acyl intermediate at the active center of the enzyme undergoes nucleophilic substitution reaction and thereby the peptide receives partial substitution and/or the like (see Fig. 2, where the peptide splicing pathway is indicated by black arrows).

Given that the peptide splicing thus includes the stage of acyl intermediate, the sooner the nucleophilic substituent attacks the acyl intermediate, which has a short half life, the more likely (absolutely more likely) the splicing reaction occurs. According to this principle, the smaller the nucleophilic substituent molecule is, the higher the reaction rate will be and the more randomly the reaction will occur.

As a candidate for the nucleophilic substituent to attack the acyl intermediate in the peptide splicing reaction, many molecular species present inside a biomolecule can be thought of. However, considering that a proteasome (an enzyme complex) has some localized enzyme activity inside its cavity such as trypsin activity, chymotrypsin activity, and peptidyl glutamyl aminopeptidase activity, and takes in a ubiquitinated protein in an active manner, the nucleophilic substituent may be limited to a peptide or an amino acid.

Thus, a molecule as the nucleophilic substituent to attack the acyl intermediate may advantageously be an amino acid and a peptide consisting of a relatively small number of residues (such as a dipeptide), most advantageously an amino acid.

Conventional spliced peptide identification uses MS/MS spectrum matching, amino acid sequence homology search, De novo sequence technique, and the like, and therefore the only peptides that can be identified are, basically, those having a recognizable local alignment. In other words, unless the peptide sequence has a sequence of three or more amino acid residues, the peptide sequence cannot be identified.

For this reason, when an amino acid and/or a dipeptide is used as a nucleophilic substituent (namely, when the number of amino acids constituting the nucleophilic substituent is one or two) in the above-described peptide splicing reaction, it is difficult to identify spliced peptides by a conventional identification approach that is based on local alignment.

In this regard, the identification method according to the present embodiment is useful, especially when identifying a spliced peptide (a variant peptide) that is highly likely obtainable through substitution and/or the like with one or two amino acid residues.

It should be noted that there has been no such spliced peptide analysis technique known to date that focuses on the fundamental mechanism of splicing reaction.

The number of residues in the amino acid sequence constituting the above-described candidate group may be from 5 to 15, for example.

Table 1 below shows a preferable example of variations for the candidate group of spliced peptides to be fed to the primary library in order for obtaining the secondary library, for an example configuration in which the parent peptide is composed of nine amino acid residues and the above-described predetermined number for residues is two. It should be noted that the parent peptide is not included in the candidate group.

In Table 1, each of N1, N2, C1, and C2 means an amino acid selected from 20 types of amino acids, and the number of variations for the peptide sequence in each row is given in the right column.

**[Table 1]**

| Substitution position | Candidate group of spliced peptides | Number of variations |
|---|---|---|
| Identified sequence (Parent peptide) | X1-X2-X3-X4-X5-X6-X7-X8-X9 | ----- |
| N-term 1 substituted | **N1**-X2-X3-X4-X5-X6-X7-X8-X9 | 20 |
| C-term 1 substituted | X1-X2-X3-X4-X5-X6-X7-X8-**C1** | 20 |
| N-term 2 substituted | **N1**-**N2**-X3-X4-X5-X6-X7-X8-X9 | 400 |
| C-term 2 substituted | X1-X2-X3-X4-X5-X6-X7-**C1**-**C2** | 400 |
| N/C-term 1 substituted | **N1**-X2-X3-X4-X5-X6-X7-X8-**C1** | 400 |
| N-term 1 added | **N1**-X1-X2-X3-X4-X5-X6-X7-X8-X9 | 20 |
| C-term 1 added | X1-X2-X3-X4-X5-X6-X7-X8-X9-**C1** | 20 |
| N/C-term 1 added | **N1**-X1-X2-X3-X4-X5-X6-X7-X8-X9-**C1** | 400 |
| N-term 2 added | **N1**-**N2**-X1-X2-X3-X4-X5-X6-X7-X8-X9 | 400 |
| C-term 2 added | X1-X2-X3-X4-X5-X6-X7-X8-X9-**C1**-**C2** | 400 |
| N-term 1 deleted | X2-X3-X4-X5-X6-X7-X8-X9 | 20 |
| N-term 2 deleted | X3-X4-X5-X6-X7-X8-X9 | 20 |
| C-term 1 deleted | X1-X2-X3 -X4-X5 -X6-X7-X8 | 20 |
| C-term 2 deleted | X1-X2-X3 -X4-X5 -X6-X7 | 20 |
| N/C-term 1 deleted | X2-X3-X4-X5-X6-X7-X8 | 20 |
| N-term 1 del/2 add | **N1**-**N2**-X2-X3-X4-X5-X6-X7-X8-X9 | 400 |
| N-term 2 del/1 add | **N1**-X3-X4-X5-X6-X7-X8-X9 | 20 |
| N-term 1 del/C-term 1 add | X2-X3-X4-X5-X6-X7-X8-X9-**C1** | 20 |
| N-term 1 del/C-term 2 add | X2-X3-X4-X5-X6-X7-X8-X9-**C1**-**C2** | 400 |
| N-term 2 del/C-term 1 add | X3-X4-X5-X6-X7-X8-X9-**C1** | 20 |
| N-term 2 del/C-term 2 add | X3-X4-X5-X6-X7-X8-X9-**C1**-**C2** | 400 |
| C-term 1 del/2 add | X1-X2-X3-X4-X5-X6-X7-X8-**C1**-**C2** | 400 |
| C-term 2 del/1 add | X1-X2-X3-X4-X5-X6-X7-**C1** | 20 |
| C-term 1 del/N-term 1 add | **N1**-X1-X2-X3-X4-X5-X6-X7-X8 | 20 |
| C-term 1 del/N-term 2 add | **N1**-**N2**-X1-X2-X3-X4-X5-X6-X7-X8 | 400 |
| C-term 2 del/N-term 1 add | **N1**-X1-X2-X3-X4-X5-X6-X7 | 20 |
| C-term 2 del/N-term 2 add | **N1**-**N2**-X1-X2-X3-X4-X5-X6-X7 | 400 |
| N/C-term 1 del/C-term 1 add | X2-X3-X4-X5-X6-X7-X8-**C1** | 20 |
| N/C-term 1 del/C-term 2 add | X2-X3-X4-X5-X6-X7-X8-**C1**-**C2** | 400 |
| N/C-term 1 del/N-term 1 add | **N1**-X2-X3-X4-X5-X6-X7-X8 | 20 |
| N/C-term 1 del/N-term 2 add | **N1**-**N2**-X2-X3-X4-X5-X6-X7-X8 | 400 |

### (Fourth Step: S4)

In the fourth step (S4) according to the present embodiment, the secondary library is searched for an amino acid sequence that matches the mass spectrometry data, to identify a spliced peptide contained in the sample.

An example of the specific procedure in the third step and the fourth step is as follows. For the peptide in the list (the parent peptide in the list) identified by database searching, new sequences are randomly generated based on substitution and the like of one or two amino acid residues at both termini to create an FASTA file. The resulting FASTA file (the secondary library) is reconstructed by Mascot Server (Matrix Science), followed by another round of search to identify a spliced peptide.

The above description is about a method involving randomly generating sequences from an identified peptide and performing another round of search, but, it is expected that the future development of software and the like (like Error tolerant search) would make it sufficiently possible to develop a sequence analysis technique that enables virtually constructing sequences and performing another round of search.

### (Selecting Peptide Vaccine for Cancer Vaccine Therapy)

In the seeking for cancer neoantigens, the ultimate goal is to develop and evaluate an effective cancer vaccine and cell therapy, for example.

In view of the properties of cancer, an attenuated vaccine such as a virus cannot be used as a cancer vaccine candidate. So, attempts were made using traditional cancer antigens (molecules overexpressed in cancer), oncogenes (genes that tend to have cancer-specific mutation accumulated therein), and the like as a candidate for a cancer vaccine, but, unfortunately, those attempts were less than successful.

It is desirable that current best novel cancer vaccines be focused on fragments containing oncogene mutation, proteins derived from incomplete translation, spliced peptides, and the like; and it is also desirable to use the information on HLA-binding peptides presented by them, in combination with assay results such as those from individual HLA typing (use of a peptide vaccine having a high affinity for an HLA allele expressed in individual HLA type) and microsatellite instability, to implement personalized medicine administering a peptide vaccine in a selective manner. In this regard, the identification method according to the present embodiment is applicable to efficient searching for a spliced peptide as a cancer antigen.

Many machine-learning-based predictive indicators are developed for identifying an immunogenic T cell epitope based on the binding affinity for main MHC (HLA) classes, Class I and Class II. A tool that can be used for predicting HLA affinity and the like may be any of various known ones, such as, for example, NetMHCpan (DTU Health Tech, Center for Biological Sequence Analysis, Bioinformatic unit, Technical University of Denmark), Mascot proteome server (Matrix Sciences) and PEAKS X (Bioinformatics Solutions).

A protein amino acid sequence database that can be used for the identification in the identification method according to the present embodiment may be a public database, which however can be replaced, without a great problem, by individual cancer clinical sequence information. It is expected that this makes it possible to seek for an HLA-binding peptide in a direct association with cancer-patient-specific genetic information, and also allows for sufficiently applying the identification method according to the present embodiment to a new medical care compatible with personalized medicine such as evaluation and monitoring of novel cancer vaccines, development of viral vaccines, evaluation of drug efficacy and safety, and immune monitoring.

Moreover, the identification method according to the present embodiment makes it possible to efficiently detect a spliced fragment of a random HLA-binding peptide having no predictable local alignment, and thereby may make possible to seek for (or identify) a novel cancer antigen (cancer neoantigen).

### EXAMPLES

Next, a more detailed description will be given of the present invention referring to Examples, which are not intended to limit the scope of the present invention. In the below description, an amino acid (residue) may be expressed with a three-letter abbreviation.

### <Reference Example 1>

In Vitro Test for Surveying Spliced Peptide Generated during Proteasome Reaction

### (i) Proteasome Reaction (Peptide Splicing Reaction)

20S Immunoproteasome (15 nM, R&D Systems), PA28α Activator (150 nM, R&D Systems), a mixture of 20 types of amino acids (concentration of each amino acid, 25 µM; Cambridge Isotope and Sigma Aldrich), and a proteasome substrate (1 µM of a mixture of eight types of substrates listed below in equal proportions) were brought into reaction in a 25-mM Tris-HCl buffer at 37°C for five hours or overnight. The reaction was terminated by addition of FA (final concentration, 1 mass%) and ACN (final concentration, 10 mass %).

As the proteasome substrate (a peptide to be degraded by proteasome reaction), a mixture of the following eight types of substrates (S-220, S-230, S-260, S-280, S-300, S-310, S-320, and S-330; R&D Systems) in equal proportions was used.
S-220: Z-LLL-AMC
S-230: Z-LLE-AMC
S-260: Suc-LY-AMC
S-280: Suc-LLVY-AMC
S-300: Boc-LRR-AMC
S-310: Ac-PAL-AMC
S-320: Ac-ANW-AMC
S-330: Ac-WLA-AMC

In the formula at right of each entry, the C-terminal AMC (7-amino-4-methylcoumarin) is an aminocoumarin fluorescent chromophore, which can be freed by hydrolysis of the substrate and monitored at fluorescence emission wavelength (Em) 345 nm/excitation wavelength (Ex) 445 nm.

As for the N-terminus, Z denotes a benzoyl group; Suc denotes a succinyl group; Boc denotes a tert-butoxycarbonyl group; and Ac denotes an acetyl group. Each of Z, Suc, Boc, and Ac is a protecting group and may be abbreviated as PG in the below description.

Other letters are one-letter amino acid abbreviations (for example, L denotes leucine, and E denotes glutamic acid).

### (ii) Mass Spectrometry of Peptides Contained in Proteasome Reaction Fluid

For each of the substrates (peptides), a reaction in which the C-terminal AMC was freed by hydrolysis and then an amino acid was added to the C-terminus of the peptide was monitored by multiple reaction monitoring (MRM). Optimum MRM conditions were selected for each substrate.

After the proteasome reaction described above, mass spectrometry of each peptide contained in the reaction fluid was carried out by the above-described MRM using a liquid chromatograph mass spectrometer (LC-MS) (LCMS-8050, manufactured by Shimadzu Corporation). The transitions to be detected by LC-MS were substrate PG-AAs-AMC (PG=protecting group, AAs=amino acids), free chromophore Free AMC, substrate hydrolyzed product PG-AAs-COOH, and spliced product PG-AAs-X-COOH (X denotes a random amino acid residue).

The LCMS-8050 analysis conditions were as follows:
Solvent A: Solvent containing 0.1 mass% FA with the remainder being made up of water
Solvent B: Solvent of 0.1 mass% FA and 100 mass% ACN
Separation column: Shimpack GISS, 2 µm, 2 × 50 mm
Flow rate: 0.4 mL/min
Interface: ESI interface
Transition time: 10 msec
Collision gas pressure: 270 kPa
Interface temperature: 300°C
DL temperature: 250°C
Heat block temperature: 400°C
Nebulizer gas: 3 L/min
Heating gas: 10 L/min
Drying gas: 10 L/min
Interface voltage: 4 kV

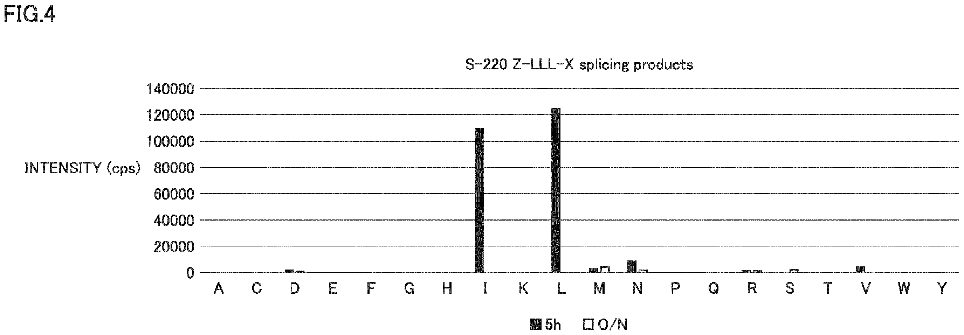

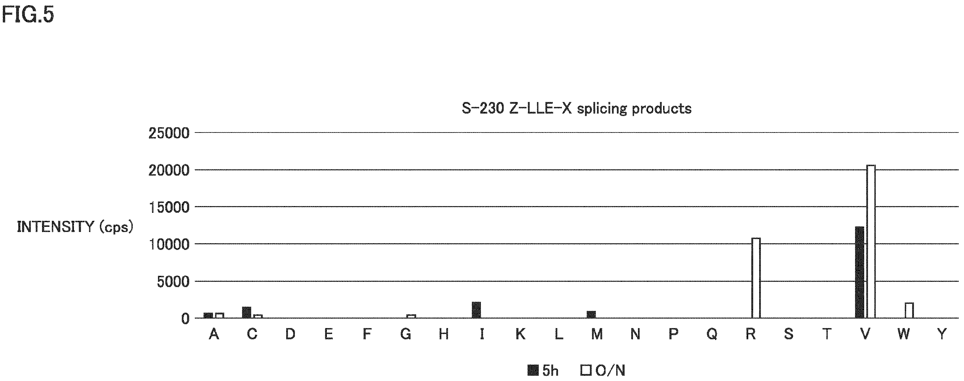

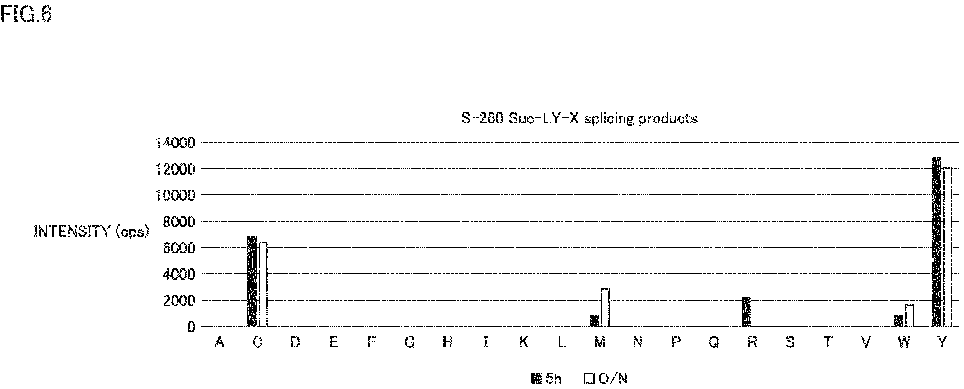

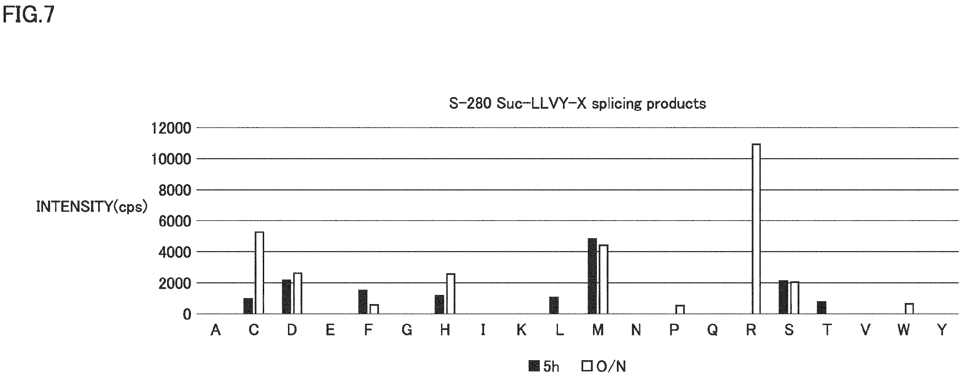

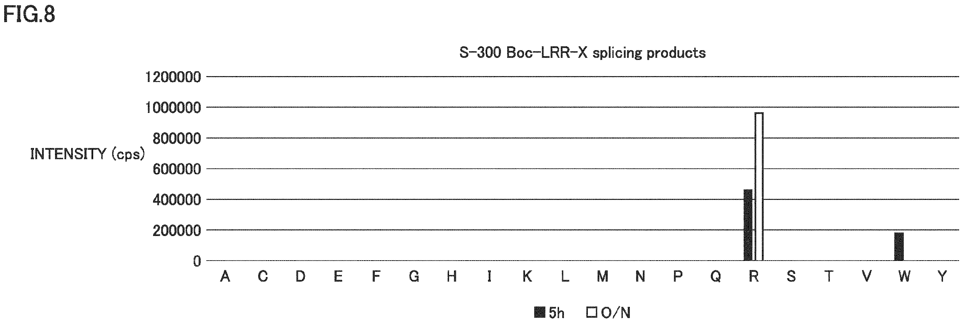

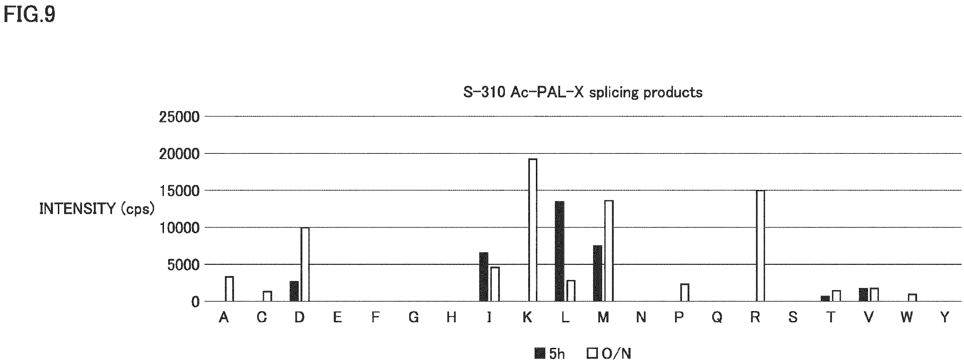

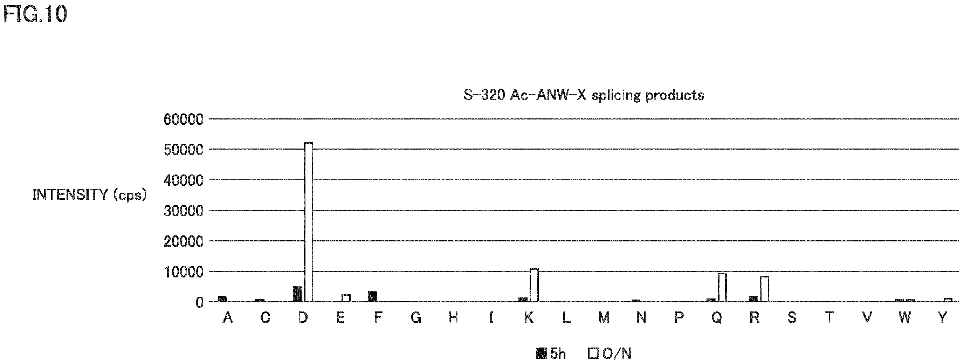

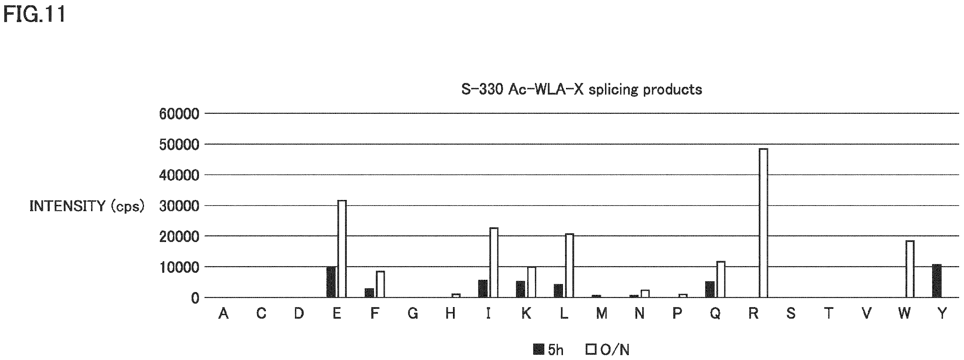

Thus, the sequences of peptides that were actually produced by splicing reaction of each of the eight types of proteasome substrates (S-220, S-230, S-260, S-280, S-300, S-310, S-320, and S-330) were checked. Analysis results obtained for the substrates are shown in Figs. 4 to 11, respectively.

In Figs. 4 to 11, the vertical axis represents the intensity, which is a value of intensity (unit, cps) measured for an MRM transition for each amino acid (one-letter abbreviation) on the horizontal axis corresponding to X (N-terminal amino acid residue) of spliced product PG-AAs-X-COOH (X denotes a random amino acid residue). In each figure, the black bar shows the value obtained when the proteasome reaction time was 5 hours (5 h), and the white bar shows the value obtained when the proteasome reaction time was overnight (O/N).

From the results shown in Figs. 4 to 11, production of spliced peptides by proteasomes was examined.

### <Example 1>

Prior to the identification method according to this Example, the below preparation was carried out.

### (Preparation of W6/32 Antibody)

(i) Mouse hybridoma W6/32 cells (ATCC: American Type Culture Collection) were cultured in RPMI1640 synthetic medium (Sigma Aldrich) containing 10 mass% FBS (Fetal Bovine Serum, Gibco). The mouse hybridoma W6/32 cell is a cell that produces W6/32 antibody (IgG2a) capable of binding to HLA-A, HLA-B, and HLA-C.
(ii) When 70% confluence was reached, the cells were rinsed for FBS removal, and the medium was changed to a serum-free hybridoma medium (Hybrigro, Corning).
(iii) Culture was continued for 72 hours.
(iv) The medium was collected, followed by filtration and cleaning.
(v) W6/32 antibody contained in the medium was made to adsorb on Protein A Sepharose (GE) column for purification, and the column was rinsed, immediately followed by elution of W6/32 antibody with 100-mM Glycine-HCl buffer (pH2.7).
(vi) Immediately after this, the pH of the solution containing the eluted W6/32 antibody was made neutral with 1-M Tris-HCl buffer (pH9.0), followed by buffer change to 200-mM Na Phosphate buffer (pH7.0).
(vii) The protein concentration of the resulting solution was measured by bicinchoninic acid assay, and the solution containing W6/32 antibody was stored at 4°C.

### (Preparation of Biological Sample)

(i) 2×10⁸ A431 cells derived from Homo sapiens epidermoid carcinoma (ATCC) were suspended in 100-mM Tris-HCl buffer (pH8.5) containing 2 mass% OTG (octyl-D-1-thioglucopyranoside) and Protease inhibitor cocktail (Sigma Aldrich), followed by incubation on ice for 30 minutes for cell lysis.
(ii) The liquid containing the lysed cells was centrifuged (20000 g, 30 min), and the supernatant was collected. It was expected that the supernatant contained HLA (HLA-A, HLA-B, and HLA-C) bonded to a characteristic peptide of A431 cells.
(iii) 200 µg of W6/32 antibody was added to the supernatant, followed by incubation at 4°C for 16 hours to form an immune complex of the W6/32 antibody and the HLA (each of HLA-A, HLA-B, and HLA-C) bonded to a characteristic peptide of A431 cells (antigenic peptide-HLA-W6/32 complex).
(iv) A resin called "TOYOPEARL AF-rProteinA" having immobilized Protein A capable of adsorbing IgG (TOSOH) was used to collect the antigenic peptide-HLA-W6/32 complex.
(v) The Protein-A-immobilized resin was rinsed five times with PBS (phosphate-buffered saline) in an amount of 1 mL each time, and then rinsed another five times with Tris buffer in an amount of 1 mL each time.
(vi) After the rinsing, 500 µL of 10% acetic acid was added to the Protein-A-immobilized resin, followed by incubation at room temperature for 10 minutes, and then the supernatant containing the antigenic peptide-HLA-W6/32 complex was collected and thoroughly dried in a centrifugation dryer. Thus, a solid containing the antigenic peptide-HLA-W6/32 complex was obtained.
(vii) The resulting solid was redissolved in 0.1% formic acid in water.

The resulting solution containing the antigenic peptide-HLA-W6/32 complex was used as a mass spectrometry sample (a biological sample).

### [Identification of Spliced Peptide]

Next, a description will be given of the identification method according to this Example, more specifically, a method of identifying an unknown spliced peptide (an unknown antigenic peptide, neoantigen) derived from a known parent peptide contained in the above biological sample.

### (1) Mass Spectrometry (the first step according to the above embodiment)

For use as the mass spectrometry data regarding a peptide contained in the sample, an MS/MS spectrum was obtained by LC-MS/MS measurement using a liquid chromatograph mass spectrometer system (Nexera-Mikros, Shimadzu Corporation) and a Q-TOF mass spectrometer (LCMS-9030, Shimadzu Corporation).

The LC-MS/MS analysis conditions were as follows:
Solvent A: Solvent containing 0.1 mass% FA (formic acid) and 5 mass% ACN (acetonitrile) with the remainder being made up of water
Solvent B: Solvent containing 0.1 mass% FA (formic acid) and 80 mass% ACN (acetonitrile) with the remainder being made up of water
Trap column: "L-column2 ODS" (Chemicals Evaluation and Research Institute, Japan), 5 µm, 0.3 × 5 mm
Separation column: "L-column2 ODS", 2 µm, 0.3 × 150 mm
Flow rate: 5 µL/min
Interface: ESI microinterface
Number of events: 8 to 14
Event duration: 100 msec
Collision voltage: 25±10 V
Collision gas pressure: 230 kPa
Interface voltage: 3 kV
Interface temperature: 100°C
DL temperature: 200°C
Heat block temperature: 250°C
Scanning range: 400 to 700 Da
Ions with undetermined valence: Excluded
Parent ion resolving power: 20 ppm
Exclusion time: 5 sec
Nebulizer gas: 1 L/min
Heating gas: 3 L/min
Drying gas: 0
Data conversion: mzML format

### (2) Identification of Parent Peptide Contained in Sample (the second step according to the above embodiment)

The mzML-format file of MS/MS spectra thus obtained was used to carry out normal Peptidome analysis to identify a known HLA Class I-binding peptide (parent peptide) as a cancer antigen peptide among the peptides contained in the sample. In the Peptidome analysis, the identification engines used were "Mascot Proteome Server version 2.6.2" (Matrix Science) and "Peaks Studio software version 10.0" (Bioinformatics Solutions Inc.), and the public protein database (amino acid sequence database) used was "SwissProt Human protein sequence version 2019.9". The SwissProt database (primary library) was searched, and a peptide that had a significant peptide score (P<0.05) according to the identification engines was identified as a parent peptide. The SwissProt database is not a peptide ligand database; it is a public protein sequence data.

### (3) Creation of Secondary Library (the third step according to the above embodiment)

A list of the identified parent peptides was exported. Random sequences (a candidate group of spliced peptides) of these parent peptides (631 types) were generated, and this candidate group was used as the secondary library.

### (4) Identification of Spliced Peptide (the fourth step according to the above embodiment)

The resulting secondary library was used to re-analyze the above mass spectrometry data (MS/MS spectrum) (to identify a spliced peptide contained in the sample).

Table 2 shows results from the peptide identification in this step. Referring to the left columns in Table 2, the results from the peptide identification are divided into Addition, Deletion, Deletion and Addition, and Substitution, each representing how the individual candidate group of spliced peptides of the secondary library could be generated from the parent peptide.

In the Table, the column "Identified peptides" shows the total number of identified peptides, and column "Identified spliced peptides" shows the number of peptides identified as spliced peptides (amino acid sequences that were not in the primary library, but were fed to the secondary library) among the identified peptides (the same applies to Table 3 below).

**[Table 2]**

| Sequence data | | Identified peptides | Identified spliced peptides |
|---|---|---|---|
| Secondary library | Addition | 567 | 32 |
| | Deletion | 584 | 17 |
| | Deletion and Addition | 630 | 134 |
| | Substitution | 637 | 332 |
| Primary library | (SwissProt protein seq) | 631 | 0 |

### <Reference Example 2>

A feasibility study was carried out on a spliced peptide identification technique using the same mass spectrometry data as in Example 1.

To start, as a database of known amino acid sequences, a primary library consisting of sequences of ligands (1231612 entries) accumulated in IEDB (Immune Epitope Database) and sequences of ligands (64201 entries) accumulated in HLAtlas was used, and splicing frequency was analyzed.

More specific procedure is as follows. For all the above sequences, random sequences (a candidate group of spliced peptides) with substitution or addition as specified in the second row and lower in Table 3 were generated, which were then fed to the original FASTA file to create a re-organized amino acid sequence database (a secondary library). From the resulting secondary library, an amino acid sequence that matches the mass spectrometry data for each peptide contained in the proteasome reaction fluid was identified. Results are shown in Table 3.

For the feasibility study, results regarding "SwissProt protein seq" are also shown.

**[Table 3]**

| Sequence data | | Identified peptides | Identified spliced peptides |
|---|---|---|---|
| Primary library | IEDB/HLAtlas ligand | 544 | 0 |
| Secondary library | N-term 1 substituted | 434 | 16 |
| | C-term 1 substituted | 461 | 10 |
| | N-term 2 substituted | 535 | 96 |
| | C-term 2 substituted | 523 | 31 |
| | N/C-term 1 substituted | 329 | 1 |
| | N-term 1 added | 288 | 17 |
| | C-term 1 added | 313 | 17 |
| | N/C-term 1 added | 3 | 3 |
| | (SwissProt protein seq) | 631 | 0 |

The results in Table 3 show that a spliced peptide can be identified at about 5% frequency on average. However, an enormous amount of time (about 10 to 30 days) and data (about 3 to 10 TB) was required for generating random sequences to create the secondary library, because the candidate group was created so as to include spliced peptides obtainable from all the sequences in the primary library. So, it is considered to be difficult to put the above method (which uses IEDB/HLAtlas database) into practical use with ordinary hardware performance.

In contrast to this, in Example 1, a known peptide (a parent peptide) contained in the sample was identified using the primary library, and then only a list of spliced peptides obtainable from the identified parent peptide (a candidate group) was fed to create the secondary library. This enabled the database size of the secondary library to be as compact as about 300 MB and the operation time to be significantly reduced (by about 30 minutes) in Example 1. The creation of the secondary library can be automated, and therefore a further reduction in processing time can be expected. Then, the resulting level will be sufficient for practical use.

### <Example 2> Predictive Analysis on Affinity for HLA

Although the spliced peptide identification in Example 1 indicated that it was likely that the mass spectrometry data matched the structure of a virtual spliced peptide derived from an HLA-binding peptide, the affinity of the identified spliced peptide for HLA was not clear. So, in this Example, the list of spliced peptides identified in Example 1 was put on the NetMHCpan algorithm, and the predicted affinity for HLA allele (HLA-A03:01, HLA-B07:02, HLA-C07:02) expressed by A431 cells was calculated.

Calculation results were used to create a score-based ranking of the spliced peptides identified in Example 1, where a case with "%Rank score" of lower than 0.5 was rated as strongly bonded ("S" in Table) and a case with "%Rank score" of not lower than 0.5 and lower than 2 was rated as weakly bonded ("W" in Table). Table 4 lists the rating results (the total number of peptides rated as S and W).

**[Table 4]**

| Secondary library | HLA-A03:01 | HLA-B07:02 | HLA-C07:02 |
|---|---|---|---|
| Addition | S: 10 | S: 6 | S: 1 |
| | W: 1 | W: 8 | W: 3 |
| Deletion | S: 5 | S: 7 | S: 0 |
| | W: 1 | W: 1 | W: 1 |
| Deletion and Addition | S: 21 | S: 27 | S: 1 |
| | W: 17 | W: 12 | W: 9 |
| Substitution | S: 36 | S: 20 | S: 5 |
| | W: 44 | W: 12 | W: 16 |

Based on the results of S (strongly bonded) rating in Table 4, about 140 spliced peptides were identified as having potential to be capable of strongly binding to HLA (HLA-A03:01, HLA-B07:02, HLA-C07:02) expressed by A431 cells.

The above-obtained analysis data on HLA affinity can enable application of a newly identified spliced peptide (a neoantigen) as a peptide vaccine for cancer vaccine therapy.

Moreover, the above-obtained data on affinity for each HLA allele can enable selecting a spliced peptide (a neoantigen) with a high affinity for an HLA allele that is expressed in association with the HLA type of the individual, from spliced peptides identified by the identification method according to the above embodiment. This may be used for, for example, selecting a novel peptide vaccine candidate for cancer vaccine therapy from spliced peptides, where an optimum peptide vaccine can be selected for an individual patient based on information on a spliced peptide identified from a cancer cell and the like as well as information on the HLA type of the patient.

### [Aspects]

As will be appreciated by those skilled in the art, the example embodiments and Examples described above are specific examples of the below aspects.

### (First Item)

A method of identifying a spliced peptide according to one aspect is a method of identifying an unknown spliced peptide derived from a known parent peptide contained in a biological sample, the method comprising:
(1) a first step involving performing mass spectrometry on the sample to obtain mass spectrometry data regarding a peptide contained in the sample;
(2) a second step involving searching a primary library, which is a database of known amino acid sequences, for an amino acid sequence that matches the mass spectrometry data, to identify a parent peptide contained in the sample;
(3) a third step involving creating a secondary library including a candidate group of spliced peptides obtainable from the identified parent peptide; and
(4) a fourth step involving searching the secondary library for an amino acid sequence that matches the mass spectrometry data, to identify a spliced peptide contained in the sample.

The identification method according to the first item uses the secondary library including a candidate group of spliced peptides obtainable from the parent peptide, and thereby enables identifying unknown spliced peptides with ease and efficiency without performing, among others, proteasome degradation experiments or analysis of unidentified MS/MS spectra generated in abundance.

### (Second Item)

The identification method according to the first item, wherein the candidate group consists of spliced peptides that are highly likely obtainable from the parent peptide.

Compared to feeding the candidate group with all the variations of variant peptides obtainable from the parent peptide through splicing, the identification method according to the second item enables reducing the time for computer processing and the like for identification, and thereby allows for efficient identification.

### (Third Item)

The identification method according to the first or second item, wherein the candidate group consists of variant peptides to be obtained from the parent peptide through at least one of the following: deletion of a predetermined number or less of amino acid residues from at least one of the C-terminus and the N-terminus; and addition of a predetermined number or less of amino acid residues to the C-terminus and the N-terminus.

Compared to feeding the candidate group with all the variations of variant peptides obtainable from the parent peptide through splicing, the identification method according to the third item, in which the candidate group consists of spliced peptides that are highly likely obtainable from the parent peptide, enables reducing the time for computer processing and the like for identification, and thereby allows for efficient identification, like in the second item.

### (Fourth Item)

The identification method according to the third item, wherein the predetermined number for residues is two.

The identification method according to the fourth item, in which the candidate group consists of spliced peptides that are even more highly likely obtainable from the parent peptide, enables further reducing the time for computer processing and the like for identification, and thereby allows for further efficient identification.

### (Fifth Item)

The identification method according to any one of the first item to the forth item, wherein
the mass spectrometry data is a mass spectrometry spectrum, and
the amino acid sequence that matches the mass spectrometry data is an amino acid sequence corresponding to a mass spectrometry spectrum having a peak at the same m/z value as or at an m/z value within a predetermined deviation range from an m/z value for at least one peak in the mass spectrometry spectrum.

### (Sixth Item)

The identification method according to any one of the first item to the fifth item, wherein the parent peptide is a known HLA-binding peptide.

### (Seventh Item)

The identification method according to any one of the first item to the sixth item, wherein the parent peptide is a known HLA-binding peptide.

### (Eighth Item)

The identification method according to the seventh item, wherein the parent peptide is a known HLA-binding peptide derived from a cancer cell.

A program for implementing the above-described identification method, and a medium for storing the program (a non-transitory, computer-readable medium).

It should be construed that all the embodiments and Examples disclosed herein are given by way of illustration in all respects, not by way of limitation. It should also be construed that the scope of the present invention is interpreted by the terms of the appended claims, not by the above description, and encompasses all modifications and variations equivalent in meaning and scope to the claims.

## Claims

1. A method of identifying an unknown spliced peptide derived from a known parent peptide contained in a biological sample, the method comprising:
(1) a first step involving performing mass spectrometry on the sample to obtain mass spectrometry data regarding a peptide contained in the sample;
(2) a second step involving searching a primary library, which is a database of known amino acid sequences, for an amino acid sequence that matches the mass spectrometry data, to identify a parent peptide contained in the sample;
(3) a third step involving creating a secondary library including a candidate group of spliced peptides obtainable from the identified parent peptide; and
(4) a fourth step involving searching the secondary library for an amino acid sequence that matches the mass spectrometry data, to identify a spliced peptide contained in the sample.

2. The method according to claim 1, wherein the candidate group consists of spliced peptides that are highly likely obtainable from the parent peptide.

3. The method according to claim 1, wherein the candidate group consists of variant peptides to be obtained from the parent peptide through at least one of the following: deletion of a predetermined number or less of amino acid residues from at least one of the C-terminus and the N-terminus; and addition of a predetermined number or less of amino acid residues to the C-terminus and the N-terminus.

4. The method according to claim 3, wherein the predetermined number for residues is two.

5. The method according to claim 1, wherein
the mass spectrometry data is a mass spectrometry spectrum, and
the amino acid sequence that matches the mass spectrometry data is an amino acid sequence corresponding to a mass spectrometry spectrum having a peak at the same m/z value as or at an m/z value within a predetermined deviation range from an m/z value for at least one peak in the mass spectrometry spectrum.

6. The method according to claim 1, wherein the parent peptide is a known HLA-binding peptide.

7. The method according to claim 1, wherein the parent peptide is a known peptide derived from a cancer cell.

8. The method according to claim 1, wherein the parent peptide is a known HLA-binding peptide derived from a cancer cell.
